# EUROPÄISCHE PATENTSCHRIFT

(11) **EP 1 478 365 B1**
(45) Veröffentlichungstag und Bekanntmachung des Hinweises auf die Patenterteilung: **02.09.2009**
(21) Anmeldenummer: 03722328.6
(22) Anmeldetag: 19.02.2003
(51) Int. Cl.: A61K 31/519, A61K 31/451, A61K 31/4535, A61P 35/00, A61P 35/04, A61P 27/02, A61P 29/00, A61P 17/06, A61P 7/10, A61P 17/00

(54) **KOMBINATION VON TRAPIDIL MIT 5HT2-SEROTONINANTAGONISTEN WIE CYPROHEPTADIN ODER PIZOTIFEN ZUR HEMMUNG DER ANGIOGENESE**
COMBINATION CONSISTING OF TRAPIDIL AND 5HT2-SEROTONIN ANTAGONISTS SUCH AS CYPROHEPTADINE OR PIZOTIFEN FOR INHIBITING ANGIOGENESIS
COMBINATION DE TRAPIDIL AVEC DES 5HT2 ANTAGONISTES DE LA SEROTONINE COMME CYPROHEPTADIN OU PIZOTIFEN POUR L'INHIBITION DE L'ANGIOGENESE

(30) Priorität: 19.02.2002 DE 10206941
(43) Veröffentlichungstag der Anmeldung: 24.11.2004
(73) Patentinhaber: Nowak, Götz, 99097 Erfurt (DE)
(72) Erfinder: NOWAK, Götz, 99097 Erfurt (DE); NEUBAUER, Ines, 07616 Thalbürgel (DE)
(74) Vertreter: Vossius & Partner
(86) Internationale Anmeldenummer: PCT/EP2003/001694
(87) Internationale Veröffentlichungsnummer: WO 2003/070249

(56) Entgegenhaltungen:
- US-A- 5 272 159
- BENELLI U. ET AL.: "Trapidil inhibits endothelial cell proliferation and angiogenesis in the chick chorioallantoic membrane and in the rat cornea" JOURNAL OF OCULAR PHARMACOLOGY AND THERAPEUTICS, Bd. 11, Nr. 2, 1995, Seiten 157-166, XP009013086
- BANDO H. ET AL.: "Effects of antiplatelet agents on pulmonary metastases" GANN , Bd. 75, März 1984 (1984-03), Seiten 284-291, XP009013087

## Beschreibung

Die vorliegende Erfindung betrifft Arzneimittel enthaltend einen 5HT₂. Serotoninantagonisten in Kombination mit Tradipil.

Eine Vielzahl von Erkrankungen sind mit einer übermässigen Angiogense verbunden, hierunter sind insbesondere Tumorerkrankungen, diabetische Retinopathie, Makuladegeneration, rheumatoide Arthritis, progressive Fibrodysplasie, Psoriasis und chronisches Hirnödem als Ausdruck einer gesteigerten Gefäßpermeabilität durch exzessive Kapilarneogenese zu nennen.

Die vorliegende Erfindung stellt somit ein Arzneimittel bereit das zur Hemmung der Angiogense verwendet werden kann, insbesondere als antiangiogenetische Substanz in der Tumortherapie, zur Primärtumarprophylaxe und zur Prophylaxe von Metastasen. Weiterhin kann das erfindungsgemäße Arzneimittel bei diabetischer Retinopathie, Makuladegeneration, rheumatoider Arthritis, progressiver Fibrodysplasie, Psoriasis und chronischen Hirnödem als Ausdruck einer gesteigerten Gefäßpermeabilität durch exzessive Kapilameogenese verwendet werden.

Die Beeinflussung der Gefäßwachstumsprozesse in Tumoren bzw. die Hemmung der Gefäßaussprossung in rasch wachsenden Tumoren ist eine sehr erfolgversprechende Antitumorstrategie. In den letzten 10 - 15 Jahren wurden verschiedene Möglichkeiten einer experimentellen Hemmung der Angiogenese bzw. Kapillarneogenese entwickelt, um über diesen wichtigen Weg eine Vaskularisierung von Tumoren zu verhindern bzw. zu antagonisieren. Wesentliche Meilensteine auf diesem Gebiet war die Aufklärung der Mechanismen, die zur Tumorangiogenese führen. Es wurden die molekularbiologischen Grundlagen der Interaktion zwischen den Rezeptoren auf den vaskulären Endothelzellen und den Wachstumsfaktoren dargestellt, die als Chemokine von Tumorzellen abgegeben werden und benachbarte Vaskularitäten erreichen. Erste Erfolge wurden mit Inhibitoren von Wachstumsfaktoren bzw. mit Rezeptorantagonisten für diese Wachstumsfaktoren erzielt. Daneben wurde auch gezeigt, daß Thalidomid, ein zentral wirksames Sedativum, einen überraschend starken antiangiogenetischen Effekt aufweist. Weiterhin wurden an Angiogenese-Modellen Substanzen identifiziert, die in der Lage
sind, antiangiogenetische Effekte zu erzeugen (Udagawa T., Verheul HMW, D'Amato RJ: Thalidomide and Analogs, In: Antiangiogenic agents in Cancer Therapy. Ed.: B.A. Teicher, Humana Press Inc., 1999).

In grundlegenden Untersuchungen zur pharmakologischen Beeinflussung der Kapillarangiogenese konnte erfindungsgemäß experimentell gezeigt werden, daß vasoaktive Substanzen, die vom Serotonin abgeleitet werden können, einen Einfluß auf die Kapillarneogenese haben. Es wurde gefunden, daß vor allem 5HT₁-Agonisten die Angiogenese steigern können, im Gegensatz dazu wurden 5HT₁-Antagonisten als potentielle Inhibitoren der Kapillarneogenese identifiziert. Bei weiterem Substanzscreening wurde überraschenderweise gefunden, daß auch Cyproheptadin eine starke antiangiogenetische Wirkung entfaltet. Cyproheptadin (Peritol^{®}) wird aufgrund seiner zentralen Wirkung als Appetitsstimulanz in der Klinik verwendet. Als Nebenwirkungen waren bisher im wesentlichen sedierende Effekte bekannt. Cyproheptadin hatte sich bereits in einem experimentellen letalen Sepsismodell am Endotoxin-behandelten Läuferschwein als wirksam erwiesen (Nowak G., Markwardt F.: Thromb Haemost 53: 252-254, 1985). Es war weiterhin gefunden worden, daß Cyproheptadin die Plättchenaggregation inhibiert und die durch simultane Thrombin- und Adrenalininfusion ausgelöste Plättchen-Potenzierungsreaktion mit nachfolgendem Mikro-white-clot-Syndrom verhindert (Nowak G., Glusa E.: Folea Haematol: 116. 831-839, 1989). Die antiangiogenetische Wirkung von Cyproheptadin kann über die antiserotoninergen (5HT₂) und antiadrenergen Effekte dieser Substanz zustandekommen. Serotonin kann die Effektivität von 5HT₁-Antagonisten und Cyproheptadin auf die Angiogenesehemmung vermindern. 5HT₁-Antagonisten zusammen mit Cyproheptadin appliziert, führen zu additiven Effekten auf die Angiogenesehemmung. Zur Wirkungsanalyse wurde die Gefäßentwicklung der Dottersackmembran von befruchteten Hühnereiern benutzt. Das Modell hat den Vorteil, daß die Entwicklung dieses primären Gefäßsystems der Hühnerembryonen Kapillaren erzeugt, die eine große Ähnlichkeit mit Tumorgefäßen haben. Diese Kapillaren sind stärker fenestriert und sind anatomisch den Tumorvaskularitäten ähnlich.

In Screeninguntersuchungen identifizierten die Erfinder eine weitere Substanz, die ebenfalls eine antiangiogenetische Wirkung erzeugt und deren Wirksamkeit nicht über die Serotonin-Schiene erklärbar ist. Es handelt sich dabei um Trapidil, ein Arzneimittel zur Koronartherapie mit gefäßdilatierender Wirkung. Der eigentliche Wirkmechanismus des Trapidils auf die Koronargefäße ist in den Einzelheiten noch nicht geklärt. Überraschender Weise wurde festgestellt, daß diese Substanz einen ebenfalls sehr starken Hemmechanismus auf die Gefäßneubildung des Dottersackgefäßsystems von Hühnerembryonen zeigt. Von besonderem Interesse ist, dass überadditive synergistische Wirkungen zwischen Trapidil und Cyproheptadin im Experiment nachgewiesen werden können, bei denen die beiden Wirkungskomponenten einen sehr starken antiangiogenetischen Effekt erzeugen. In Tabelle 1 sind die überadditiven Wirkungen bei der Substanzkombination Trapidil/Cyproheptadin belegt. Werden von beiden Substanzen die IC 25-Konzentrationen gemeinsam appliziert, ist ein überadditiver Effekt von +36,6% zu messen. Ähnlich ist die optimale Kombination IC 25 Trapidil und IC 50 Cyproheptadin zu bewerten, bei der + 26% Effektivität nachweisbar waren.
Aufgrund der steilen Dosis-Wirkungs-Beziehung im Bereich von 10⁻⁷- 10⁻⁸ mol/l kann geschlußfolgert werden, daß die Wirkung in ähnlichen Dosierungen erreicht werden kann wie sie jetzt bereits für diese Substanzen in der klinischen Anwendung üblich sind.

**Tabelle 1: Kombinierte Wirkung von Cyproheptadin und Trapidil**

| | Trapidil | Kombin. | | | Kombin. | | | Kombin | | | Cyproheptadin | |
|---|---|---|---|---|---|---|---|---|---|---|---|---|
| | Hemmung | Rechner. | IC | Meßwert | Rechner | IC | Meßwert | Rechner. | IC | Meßwert | Hemmung | |
| | % | Wert | | | Wert | | | Wert | | | % | |
| | | Tr / Cy | | | Tr/Cy | | | Tr/Cy | | | | |
| IC 100 | 98,6 | 85,9 | 50/50 | 81,4 | 94,1 | 75/25 | 88,6 | 89,8 | 25/75 | 85,3 | 76,6 | IC 100 |
| | | | | (-5,2%) | | | (-5,8%) | | | (-4,8%) | | |
| IC 75 | 73,8 | 68.3 | 50/25 | 72,7 | 81,9 | 25/50 | 77,8 | | | | 65,8 | IC 75 |
| | | | | (+6,4%) | | | (+25,7%) | | | | | |
| IC 50 | 48 | 44,3 | 25/25 | 60.5 | | | | | | | 37,9 | IC 50 |
| | | | | (+35,6%) | | | | | | | | |
| IC 25 | 24 | | | | | | | | | | 20,3 | IC 25 |

In den nachfolgenden Versuchsbeispielen wird diese antiangiogenetische Wirkung gezeigt.

### Beispiel 1 - Cyproheptadin:

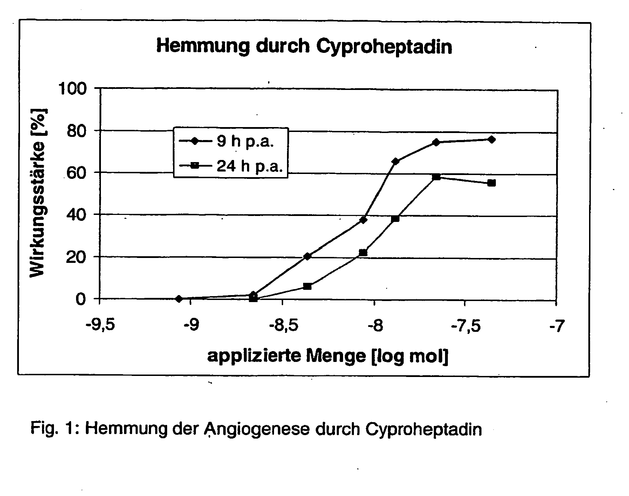

Bei 30 ex ovo bebrüteten Hühnereiern werden jeweils auf zwei Bereiche des Dottersackgefäßsystems 1,5 µl eines 3%igen Traganth-Gels, in dem Cyproheptadin in der zu untersuchenden Konzentration eingebracht wurde, appliziert. Bei weiterer Bebrütung in einem Sterilinkubator bei 37°C und 85% Luftfeuchtigkeit entwickeln sich die pre- bzw. postkapillären Gefäßstrukturen der Dottersackvaskularitäten, die 9 h und 24 h post applicationem semiquantitativ bewertet werden. In den Bereichen der Substanzapplikation kommt es zu einer deutlichen Verminderung der Vaskularisierung. Bei sehr hohen Konzentrationen ist eine totale Verhinderung einer Angiogenese nachweisbar. Die maximale Wirkungsstärke beträgt 76%. 24 h nach Applikation sind die Wirkungen in leicht abgeschwächter Form noch vorhanden (siehe Fig. 1). In Vorversuchen konnte gezeigt werden, daß die auf die Oberfläche der Dottersackmembran aufgebrachten Substanzen langsam resorbiert werden, so daß die nachgewiesene antiangiogenetische Wirkung spezifisch ist und im wesentlichen auf den Bereich der Applikationsstelle begrenzt bleibt.

### Beispiel 2- Trapidil:

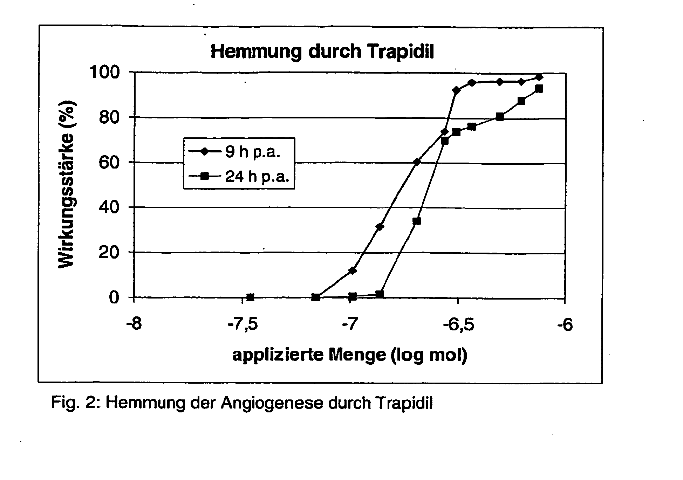

Die Applikation von Trapidil erfolgt analog der von Cyproheptadin. Ebenfalls nach 9 h und 24 h werden die Applikationsstellen bewertet. Trapidil zeigt eine deutliche Dosisabhängigkeit der Angiogenesehemmung im Bereich von 10⁻⁷ bis 10⁻⁶ mol, d.h. es besitzt eine sehr steile Dosis-Wirkungskurve. Es wird eine maximale Angiogenesehemmung von 98% erreicht. Die 24 h-Werte sind im Vergleich zu den 9h-Werten leicht verringert (siehe Fig. 2).

### Beispiel 3:

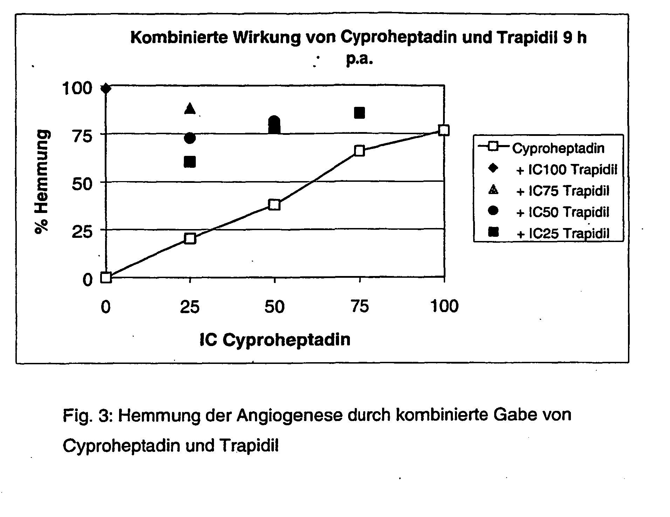

Bei kombiniertem Auftrag von Cyproheptadin und Trapidil nach dem o. g. Procedere verstärkt sich die Effektivität der Angiogenesehemmung im Vergleich zu den einzeln erzielten Wirkungen, d.h. es ist ein überadditiver Wirkmechanismus vorhanden (siehe Fig. 3, Tab. 1).

Aus diesen Untersuchungen wird somit deutlich, daß 5HT₂ Serotoninantagonisten, eine spezifische antiangiogenetische Wirksamkeit haben.

Serotoninantagonisten vom Typ 1 A hemmen hochspezifisch die Kapillarangiogenese. Cyproheptadin, ein Pharmakon mit antiserotoninerger, antiadrenerger Wirkung, hemmt hochspezifisch die Dottersackangiogenese in nanomolaren Konzentrationen.
Cyproheptadin stellt somit einen neuen Typ von Tumorhemmstoffen dar, der neben seinen spezifischen pharmakologischen Effekten auch die Kapillarneogenese hemmt und damit eine Retardierung bzw. Verhinderung des, Tumorwachstums bewirken kann.
Trapidil in Kombination mit Cyproheptadin, aber auch Trapidil allein, sind in der Lage, die Kapillarangiogenese in rasch wachsenden Kapillarstrecken vollständig zu hemmen. Durch die gemeinsame Applikation von Cyproheptadin und Trapidil ist ein überadditiver, synergistischer Effekt zu erreichen. Trapidil allein und in Kombination mit Cyproheptadin kann einen starken antiangiogenetischen Effekt erzeugen.

Die vorliegende Erfindung stellt somit neue Arzneimittel enthaltend einen 5HT₂ Serotoninantagonisten in Kombination mit Trapidil zur Hemmung der Angiogenese, insbesondere in der Tumortherapie bereit.

Erfindungsgemäß verwendete 5HT₂-Serotoninantagonisten sind z.B., in, Pizotifen, und Clozapin, sowie deren pharmazeutisch verträgliche Salze und Derivate.
Weitere bevorzugt verwendete 5HT₂-Serotoninantagonisten sind z.B. Cinanserin, Cyproheptadin, N-Desmethylclozapin, Ketaserin, Metergolinphenylmethylester, Mianserin und Spiperon, sowie deren pharmazeutisch verträgliche Salze und Derivat. Ganz besonders bevorzugt ist hierbei Cyproheptadin und Pizotifen.

Eine besonders bevorzugte Ausführungsform der vorliegenden Erfindung ist die kombinierte Anwendung von einem Cyproheptadin und Trapidil, sowie eine Kombination von Pizotifen und Trapidil ganz besonders bevorzugt.

Die erfindungsgemäß zu applizierenden Dosen des Serotoninantagonisten und-Trapidil in Kombination gegeben sind abhängig von einer Vielzahl von Faktoren, wie der individuell verwendete Verbindung, dem Gewicht, Geschlecht und Alter des zu behandelnden Patienten, der Schwere und Dauer der Erkrankung, dem Allgemeinzustand und der Begleiterkrankungen des Patienten, sowie eventueller Begleitmedikation.

Vorzugsweise liegt die orale Dosierung für den Serotoninantagonisten hierbei zwischen 0,2 mg/kg und 0,5 mg/kg Körpergewicht pro Tag. Für Trapidil liegt die orale Dosierung vorzugsweise zwischen 5 mg/kg und 9 mg/kg Körpergewicht pro Tag, bei einer Permanentinfusion bei 2-3 mg/kg/d. In Kombination liegt die Dosierung für den Serotoninantagonisten hierbei zwischen 0,1 mg/kg und 0,25 mg/kg Körpergewicht pro Tag. Für Trapidil liegt die Dosierung vorzugsweise zwischen 3 mg/kg und 6 mg/kg Körpergewicht pro Tag.

Die erfindungsgemäß verwendeten Kombinationen von 5HT₂-Serotoninantagonisten mit Trapidil (ggf. in Kombination) können in dem Fachmann an sich bekannter Weise zu Arzneimittel zur systemischen Applikation (oral oder parenteral) oder lokalen Applikation wie z.B. Tabletten, Pillen, Kapsel, Granulaten, oralen Liquida, Infusionen, Injektionen, Suppositorien, Transdermalen Therapeutischen Systemen oder Aerosolen formuliert werden. Hierzu können ggf. Hilfsstoffe wie z.B. Trägerstoffe, feste oder flüssige Verdünnungsmittel, Bindemittel, Sprengmittel, Geschmackskorrigentien und dergleichen verwendet werden.

Hierbei ist zu beachten, daß eine Kombination von einem 5HT₂-Serotoninantagonisten mit Trapidil erfindungsgemäß so verstanden wird, daß sie entweder zusammen in einer üblichen pharmazeutischen Formulierung (siehe oben) formuliert werden und somit gleichzeitig appliziert werden.

Es ist aber auch möglich, die jeweiligen Verbindungen in getrennten Formulierungen zu applizieren, und somit einen unterschiedlichen Applikationsweg (z.B. oral und parenteral) oder eine zeitversetzte Applikation zu ermöglichen.

## Patentansprüche

1. Arzneimittel enthaltend einen 5HT₂ Serotoninantagonisten in Kombination mit Trapidil als Dosierungseinheit oder zur sequenziellen Verabreichung.

2. Arzneimittel nach Anspruch 1, wobei die Serotoninar tagonisten ausgewählt sind aus der Gruppe bestehend aus Pizotifen, Clozapin, Cinaserin, Cyproheptadin, N-Desmethylclozapin, Ketaserin, Metergolinphenylmethylester, Mianserin und Spiperon, sowie deren pharmazeutisch verträgliche Salze und Derivate.

3. Arzneimittel nach Anspruch 1 oder 2, wobei Cyproheptadin oder Pizotifen in Kombination mit Trapidil verwendet wird.

4. Arzneimittel nach einem der Ansprüche 1 bis 3 zur Hemnung der Angiogenese.

5. Arzneimittel nach einem der Ansprüche 1 bis 3 als ant angionetische Substanz in der Tumortherapie.

6. Arzneimittel nach einem der Ansprüche 1 bis 3 zur Primärtumorprophylaxe.

7. Arzneimittel nach einem der Ansprüche 1 bis 3 zur Prophylaxe von Metastasen.

8. Arzneimittel nach einem der Ansprüche 1 bis 3 zur Prophylaxe oder Therapie von diabetischer Retinopathie, Makuladegeneration, rheumatoider Arthritis, progressiver Fibrodysplasie, Psoriasis und chronischem Hirnödem ab Ausdruck einer gesteigerten Gefäßpermeabilität durch exzessive Kapillarneogenese

## Claims

1. Pharmaceutical composition containing a 5HT₂ serotonin antagonist in combination with trapidile as dosage unit or for sequential administration.

2. Pharmaceutical composition according to claim 1, wherein the serotonin antagonists are selected from the group consisting of pizotifene, clozapine, cinanserin, cyproheptadine, N-desmethylclozapine, ketaserin, metergoline phenyl methyl ester, mianserine and spiperone as well as pharmaceutically acceptable salts and derivatives thereof.

3. Pharmaceutical composition according to claim 1 or 2, wherein cyproheptadine or pizotifene is used in combination with trapidile.

4. Pharmaceutical composition according to any one of claims 1 to 3 for the inhibition of angiogenesis.

5. Pharmaceutical composition according to any one of claims 1 to 3 as antiangiogenetic substance in tumour therapy.

6. Pharmaceutical composition according to any one of claims 1 to 3 for primary tumour prophylaxis.

7. Pharmaceutical composition according to any one of claims 1 to 3 for prophylaxis of metastases.

8. Pharmaceutical composition according to any one of claims 1 to 3 for prophylaxis or therapy of diabetic retinopathy, macular degeneration, rheumatoid arthritis, progressive fibrous dysplasia, psoriasis and chronic brain oedema resulting from increased vessel permeability by excessive capillary neogenesis.

## Revendications

1. Médicament contenant un antagoniste 5HT₂ de la sérotonine en combinaison avec le trapidil en tant que dose unitaire posologique ou pour une administration séquentielle.

2. Médicament selon la revendication 1, dans lequel les antagonistes de la sérotonine sont choisis dans le groupe formé par le pizotifène, la clozapine, la cinansérine, la cyproheptadine, la N-desméthylclozapine, la kétansérine, l'ester phénylméthylique de la métergoline, la miansérine, et la spipérone, ainsi que leurs sels et dérivés pharmaceutiquement acceptables.

3. Médicament selon la revendication 1 ou 2, dans lequel la cyproheptadine ou le pizotifène est employé en combinaison avec le trapidil.

4. Médicament selon l'une quelconque des revendications 1 à 3, pour inhiber l' angiogenèse.

5. Médicament selon l'une quelconque des revendications 1 à 3, en tant que substance anti-angiogénique dans la thérapie des tumeurs.

6. Médicament selon l'une quelconque des revendications 1 à 3, pour la prophylaxie des tumeurs primaires.

7. Médicament selon l'une quelconque des revendications 1 à 3, pour la prophylaxie des métastases.

8. Médicament selon l'une quelconque des revendications 1 à 3, pour la prophylaxie ou la thérapie de la rétinopathie diabétique, la dégénérescence maculaire, la polyarthrite rhumatoïde, la fibrodysplasie ossifiante progressive, le psoriasis, et l'oedème cérébral chronique en tant qu'expression d'une perméabilité vasculaire accrue par une néogenèse capillaire excessive.
